(19) Europäisches Patentamt
European Patent Office
Office européen des brevets

(11) EP 4 488 941 A1

(12) EUROPEAN PATENT APPLICATION

(43) Date of publication:
08.01.2025 Bulletin 2025/02

(21) Application number: 24181811.1

(22) Date of filing: 12.06.2024

(51) International Patent Classification (IPC):
G06T 7/62 (2017.01)          G06T 7/73 (2017.01)

(52) Cooperative Patent Classification (CPC):
G06T 7/62; G06T 7/73; G06T 2207/30196

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 03.07.2023 JP 2023109130

(71) Applicant: ASICS Corporation
Kobe-shi, Hyogo 650-8555 (JP)

(72) Inventors:
• Kitayama, Hironori
  Hyogo, 650-8555 (JP)
• Tsutsui, Masayuki
  Hyogo, 650-8555 (JP)
• Nakaya, Mai
  Hyogo, 650-8555 (JP)

(74) Representative: Marks & Clerk LLP
15 Fetter Lane
London EC4A 1BW (GB)

(54) **HEEL POSITION ESTIMATION DEVICE, HEEL POSITION ESTIMATION METHOD, AND PROGRAM**

(57) In a heel position estimation device, a contour extractor (52) extracts, from a one-foot image of a measurement subject, a contour shape of a foot part that includes at least part of a heel portion. A judgment unit (54) determines the type of the contour shape. A heel estimation unit (56) estimates a heel point position in the contour shape, based on the type of the contour shape determined.

FIG. 3

EP 4 488 941 A1

**Description**

BACKGROUND

1. Technical Field

The present disclosure relates to a heel position estimation device, a heel position estimation method, and a program.

2. Background Information

There is a known technology of measuring a foot size, such as a foot length, of a measurement subject from an image including at least one foot of the measurement subject (hereinafter, referred to as a "one-foot image"). For example, Patent Literature 1 describes a technology of acquiring, from an image capturing unit, a one-foot image obtained by capturing, when a foot is placed on a foot placement sheet at its sole and the heel end of the foot is set in line with the lower side of the foot placement sheet, an image of the entire foot placement sheet and foot. Also, in the technology, an actual length ratio is calculated based on the one-foot image by dividing the actual length of the long side of the foot placement sheet by the number of pixels on the long side of the foot placement sheet.
Patent Literature 1: Japanese Unexamined Patent Application Publication No. 2019-45463
In the technology described in Patent Literature 1, however, the measurement subject himself or herself needs to set the heel end of his or her foot in line with the lower side of the foot placement sheet, and hence, there is room for improvement in measurement accuracy. In order to accurately measure the foot length of the measurement subject, it is required to estimate the position of the heel point of the measurement subject in the one-foot image.

SUMMARY

The present disclosure has been made in view of such a situation, and a general purpose thereof is to provide a technology for enabling appropriate estimation of a heel point position from a one-foot image of a measurement subject.
A heel position estimation device according to one embodiment of the present disclosure includes: a contour extractor that extracts, from a one-foot image of a measurement subject, a contour shape of a foot part that includes at least part of a heel portion; a judgment unit that determines the type of the contour shape; and a heel estimation unit that estimates a heel point position in the contour shape, based on the type of the contour shape determined.
Another embodiment of the present disclosure relates to a heel position estimation method. This method includes: extracting, from a one-foot image of a measurement subject, a contour shape of a foot part that includes at least part of a heel portion; determining the type of the contour shape; and estimating a heel point position in the contour shape, based on the type of the contour shape determined.
Yet another embodiment of the present disclosure relates to a program. This program causes a computer to implement: extracting, from a one-foot image of a measurement subject, a contour shape of a foot part that includes at least part of a heel portion; determining the type of the contour shape; and estimating a heel point position in the contour shape, based on the type of the contour shape determined.
Optional combinations of the aforementioned constituting elements, and implementation of the present disclosure, including the constituting elements and expressions, in the form of methods, apparatuses, programs, transitory or non-transitory storage medium storing programs, or systems may also be practiced as additional modes of the present disclosure.

BRIEF DESCRIPTION OF THE DRAWINGS

Embodiments will now be described, by way of example only, with reference to the accompanying drawings which are meant to be exemplary, not limiting, and wherein like elements are numbered alike in several figures, in which:

Fig. 1 is a schematic diagram that shows an overview of a heel position estimation system;
Fig. 2 is a functional block diagram that shows a schematic configuration of the heel position estimation system shown in Fig. 1;
Fig. 3 is a functional block diagram that shows a schematic configuration of a computing unit of a heel position estimation server shown in Fig. 1;
Figs. 4A and 4B are schematic diagrams that show an example of a contour shape of a foot part extracted from a one-foot image by a contour extractor shown in Fig. 3;
Fig. 5 is a flowchart that shows an example of processing performed by the heel position estimation server shown in Fig. 1;
Fig. 6 is a flowchart that specifically shows processing performed by a judgment unit at the step S14 and processing performed by a heel estimation unit at the step S16 shown in Fig. 5, in an example of the operations performed the heel position estimation server shown in Fig. 1;
Fig. 7A shows an example of a contour shape judged to be a first type; Fig. 7B shows an example of a contour shape judged to be a second type;
Fig. 8A shows another example of a contour shape judged to be the second type; Fig. 8B shows an example of a contour shape judged to be a third type; and
Fig. 9 is a schematic diagram that shows foot length correction performed by a foot length correction unit shown in Fig. 3.

DETAILED DESCRIPTION

The invention will now be described by reference to the preferred embodiments. This does not intend to limit the scope of the present invention, but to exemplify the invention.

In the following, the present disclosure will be described based on a preferred embodiment with reference to each drawing. In the embodiment and modifications, like reference characters denote like or corresponding constituting elements, and repetitive description will be omitted as appropriate.

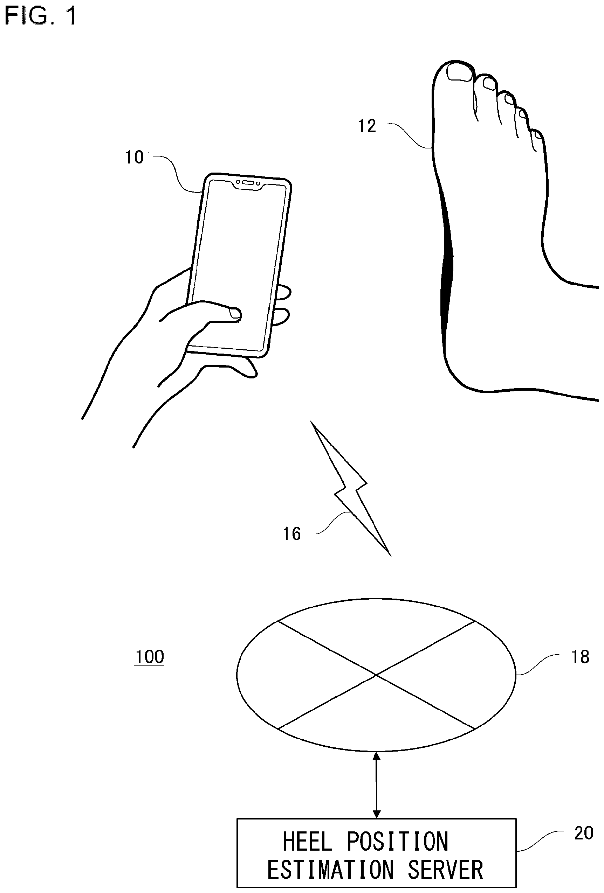

Fig. 1 is a schematic diagram that shows an overview of a heel position estimation system 100. The heel position estimation system 100 includes a user terminal 10 and a heel position estimation server 20 connected to each other via wireless communication 16 and a network 18. A measurement subject shoots an image (a one-foot image) that includes his or her own one foot 12, using a camera function of the user terminal 10. The user terminal 10 transmits the captured one-foot image to the heel position estimation server 20 via the wireless communication 16 and the network 18. The heel position estimation server 20 estimates the position of a heel point in the received one-foot image. The heel position estimation server 20 may further estimate a toe position from the received one-foot image and may calculate the foot length based on the estimated toe position and the estimated heel point position. The heel position estimation server 20 may transmit foot size information including the calculated foot length to the user terminal 10 and display the foot size information on the screen of the user terminal 10.

Each of the user terminal 10 and the heel position estimation server 20 may be constituted by a mobile terminal or a computer that includes a central processing unit (CPU), a graphics processing unit (GPU), a random access memory (RAM), a read only memory (ROM), an auxiliary storage device, a display device, a communication device, and the like, and by a program stored in the mobile terminal or computer. For example, a program executed on the heel position estimation server 20 side may be used from the user terminal 10 side via the network 18. Also, the functions of the heel position estimation system may be implemented by a stand-alone device equipped with the functions of the user terminal 10 and the heel position estimation server 20, and a measurement subject may be able to directly operate the stand-alone device to execute a heel position estimation program. The stand-alone device may be provided in the form of, besides a personal computer, a mobile terminal, such as a smartphone, or a tablet terminal, along with a program stored in the computer or terminal. Alternatively, the device may be implemented in the form of a terminal installed in a shoe store and operated by an assistant such as a salesperson, along with a program stored in the terminal.

The "heel position estimation device" in the present specification may mean the entire heel position estimation system 100 or may mean the heel position estimation server 20. In the present embodiment, the heel position estimation server 20 substantially corresponds to the "heel position estimation device" because many of the characteristic functions included in the "heel position estimation device" are implemented such as to be included in the heel position estimation server 20. The characteristic functions of the "heel position estimation device" may also be distributed between the user terminal 10 and the heel position estimation server 20 or may also be implemented such that many of them are included in the user terminal 10.

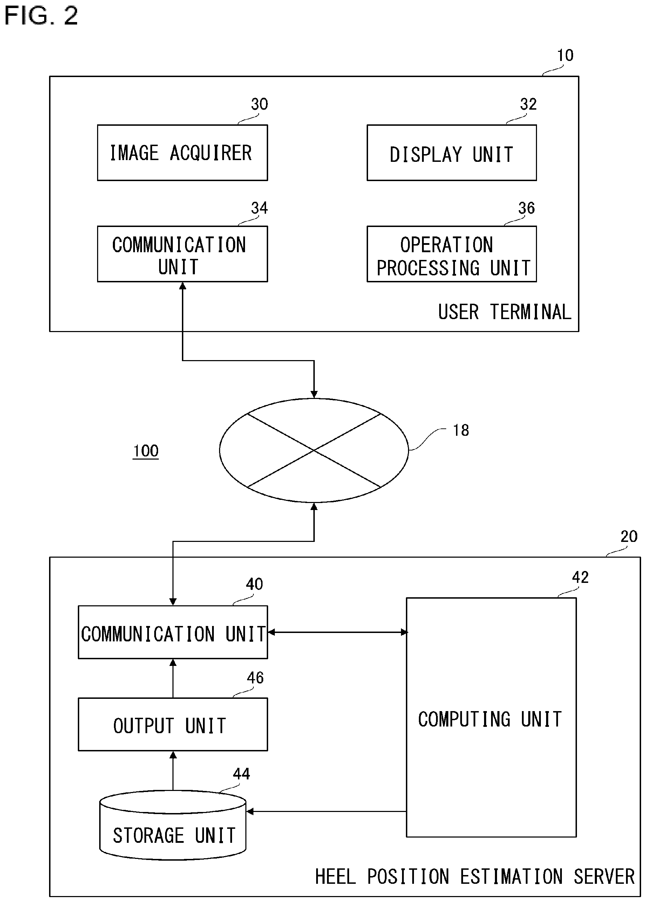

Fig. 2 is a functional block diagram that shows a schematic configuration of the heel position estimation system 100. Each of the functional blocks shown in the diagrams, including Fig. 2, can be implemented by an element such as a processor or memory of a computer or by a mechanism in terms of hardware, and by a computer program or the like in terms of software. Fig. 2 illustrates functional blocks implemented by coordination of those components. These functional blocks may be implemented in a variety of forms by hardware only, software only, or a combination thereof.

The user terminal 10 includes an image acquirer 30, a display unit 32, a communication unit 34, and an operation processing unit 36. The operation processing unit 36 accepts operation input from a measurement subject. The operation processing unit 36 accepts, from the measurement subject, an instruction regarding the start of a series of foot size measurement programs including the heel position estimation program. Based on the instruction accepted by the operation processing unit 36, the display unit 32 starts screen display of the contents of the foot size measurement programs and displays contents related to the measurement procedure. The operation processing unit 36 and the display unit 32 may be constituted by a touch panel, for example, in terms of hardware.

The image acquirer 30 captures an image of one foot of the measurement subject to acquire a one-foot image. The one-foot image is an image of a region including at least the heel point of the one foot 12. When the heel position estimation server 20 includes a toe estimation unit 58, which will be described later, the one-foot image is an image of a region further including at least the toe of the one foot 12. The one-foot image may be an image that can be captured by the measurement subject himself or herself by operating the user terminal 10. In this case, as shown in Fig. 1, for example, the measurement subject can use, as the one-foot image, an image of his or her own one foot 12 shot from diagonally above on the medial side. The one-foot image may be an image of the one foot 12 shot from diagonally above on the lateral side.

The one-foot image may be captured to include the one foot 12 together with a predetermined reference object, as described below. The "reference object" is an object to be captured together with one foot in the image, as a comparison object used to measure the foot length or the foot width of the one foot based on the one-foot image, and is a planar or three-dimensional object of which the size and shape are predetermined. The "reference object" may be a general-purpose product as long as the size and shape thereof are determined or may be an exclusive product of which the size and shape are determined exclusively for the measurement in the present embodiment. As a reference object in the present embodiment, a planar object of predetermined size and shape, such as A4 size (210 mm x 297 mm) paper, may be used. The color of the paper may preferably be white but may be another color. The one-foot image in the present embodiment is an image obtained by shooting one foot along with the entire A4 white paper as the reference object on which the one foot is placed. It is premised that the A4 white paper is placed on a non-white floor during a shoot, so that the A4 white paper can be accurately extracted from the one-foot image by image recognition. The one-foot image may be an image that includes other objects besides the one foot 12 of a measurement subject and the reference object, such as part of the other foot of the measurement subject.

The image acquirer 30 may be constituted by a camera module, for example, in terms of hardware. The communication unit 34 transmits the one-foot image to the heel position estimation server 20 via the network 18 and receives the foot size information from the heel position estimation server 20 to transmit the information to the display unit 32. The communication unit 34 may be constituted by a wireless communication module for wireless LAN communication or cellular phone communication, for example, in terms of hardware. The display unit 32 displays, on the screen, the one-foot image and the foot size information of the measurement subject.

The heel position estimation server 20 includes a communication unit 40, a computing unit 42, a storage unit 44, and an output unit 46. The communication unit 40 receives a one-foot image from the user terminal 10 and transmits the computation results from the computing unit 42, such as the foot size information, to the user terminal 10. The communication unit 40 may be constituted by a communication module for a wired LAN, for example, in terms of hardware.

The computing unit 42 performs computing processing including the process of estimating the heel point position, based on a one-foot image received by the communication unit 40. The computing processing performed by the computing unit 42 will be detailed later.

The computing unit 42 stores the computation results in the storage unit 44. The computation results stored in the storage unit 44 are transmitted from the output unit 46 to the user terminal 10 via the communication unit 40.

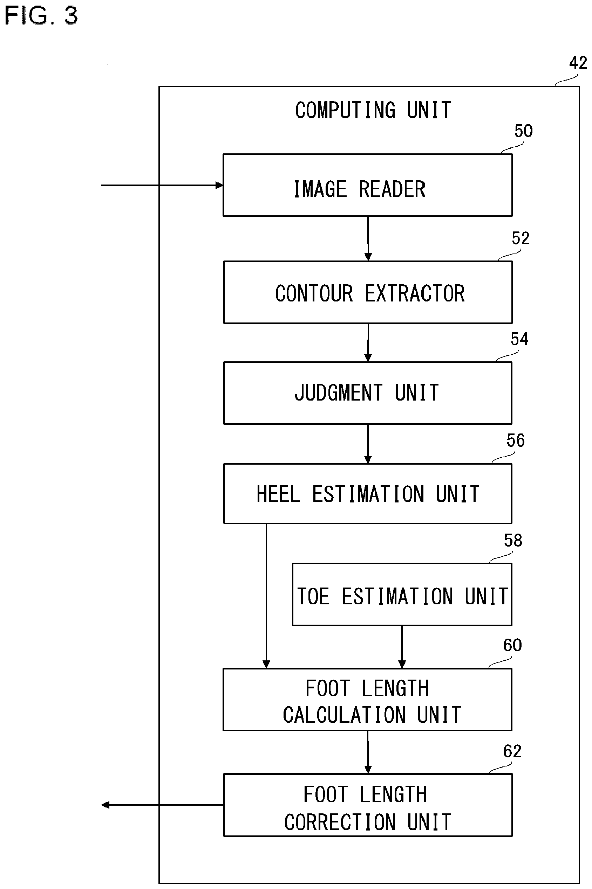

Fig. 3 is a functional block diagram that shows a schematic configuration of the computing unit 42 of the heel position estimation server 20. The computing unit 42 includes an image reader 50, a contour extractor 52, a judgment unit 54, and a heel estimation unit 56.

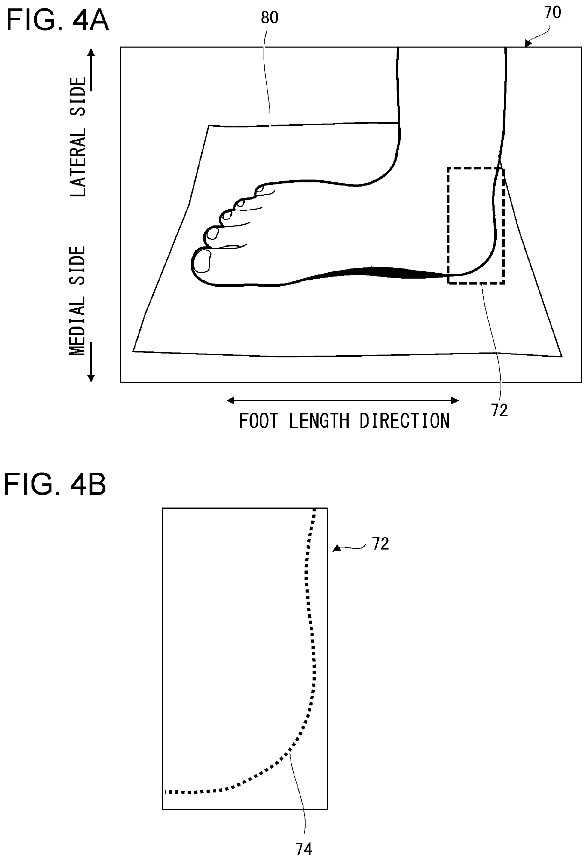

The image reader 50 reads a one-foot image received by the communication unit 40 from the user terminal 10. From a one-foot image of a measurement subject read by the image reader 50, the contour extractor 52 extracts a contour shape of a foot part that includes at least part of a heel portion. Figs. 4A and 4B are schematic diagrams that show an example of a contour shape 74 of a foot part extracted by the contour extractor 52 from a one-foot image 70.

Fig. 4A is a schematic diagram that shows the position of a foot part of which the contour shape 74 is extracted in the one-foot image 70. In the example shown in Fig. 4A, the one-foot image 70 is a two-dimensional image captured from diagonally above on the medial side such that a one foot placed on white paper 80 as the reference object is entirely captured. As will be described later, the heel estimation unit 56 estimates one point on the contour shape 74 extracted by the contour extractor 52 from the one-foot image 70, to be the heel point position. Therefore, the one-foot image 70 is an image captured from the medial side or lateral side of one foot such that the heel point is present on the contour of the captured one foot.

In the one-foot image 70, a region corresponding to a foot part of which the contour shape 74 is extracted is indicated as a cut-out region 72. The cut-out region 72 includes at least part of the heel portion but may not include the entire heel portion. The cut-out region 72 may also include a portion of the one foot other than the heel portion. However, for the estimation of the heel point position by the heel estimation unit 56, the cut-out region 72 needs to include the heel point. As will be described later, the heel estimation unit 56 estimates the heel point position based on the type of the contour shape 74 determined by the judgment unit 54. Accordingly, at the time when the contour extractor 52 extracts the contour shape 74, the estimated heel point position is unknown. Therefore, the contour extractor 52 needs to set, as the cut-out region 72, a region that includes all possible heel point positions to be estimated no matter which type the contour shape 74 is judged to be. The cut-out region 72 may be set based on a predetermined algorithm, such as pattern recognition. The shape of the cut-out region 72 is not limited to a rectangle as shown in Fig. 4A and may be an arbitrary shape.

Fig. 4B is a schematic diagram that shows an example of the contour shape 74 extracted by the contour extractor 52. The contour extractor 52 extracts the contour shape 74 by detecting the contour of one foot shown in the one-foot image 70 and identifying a shape along the contour thus detected. For example, by binarizing the cut-out region 72, the contour extractor 52 can detect the contour between the region occupied by the one foot and the region occupied by the white paper 80 in the cut-out region 72. Along the contour thus detected, the contour extractor 52 extracts the contour shape 74. The data of the contour shape 74 may be in the raster format in which the contour shape 74 is represented by a set of points in a two-dimensional coordinate system, or in the vector format in which the contour shape 74 is represented by a set of lines in a two-dimensional coordinate system. When the data of the contour shape 74 is in the raster format as shown in Fig. 4B, the interval between points constituting the contour shape 74 may be arbitrary, and the number of points may vary depending on the shape of the contour shape 74 included in the cut-out region 72. When extracting the contour shape 74, the contour extractor 52 may perform an arbitrary smoothing process to remove noise from the one-foot image 70, for example.

As described above, the contour shape 74 thus extracted is two-dimensional information extracted from the one-foot image 70. Even if the same one foot of the same measurement subject is captured, the one-foot image 70 may differ depending on the capturing direction or capturing angle. In addition, there are individual differences in the three-dimensional shape of one foot of the measurement subject. Thus, the contour shape 74 may vary depending on the capturing direction or capturing angle of the one-foot image 70 and the individual differences in the three-dimensional shape of one foot of the measurement subject.

Referring back to Fig. 3, the judgment unit 54 determines the type of the contour shape extracted by the contour extractor 52. The judgment processing performed by the judgment unit 54 will be detailed later.

Based on the type of the contour shape determined by the judgment unit 54, the heel estimation unit 56 estimates the heel point position in the contour shape. The estimation processing performed by the heel estimation unit 56 will be detailed later.

In this way, the heel position estimation server 20 of the present embodiment determines the type of the contour shape 74 using the judgment unit 54 and estimates the heel point position based on the type of the contour shape 74 using the heel estimation unit 56. Therefore, the heel position estimation server 20 can reduce the influence of the capturing conditions of the one-foot image 70 or the individual differences in the three-dimensional shape of one foot and can improve the accuracy of estimating the heel point position.

As shown in Fig. 3, the computing unit 42 of the heel position estimation server 20 may further include the toe estimation unit 58, a foot length calculation unit 60, and a foot length correction unit 62.

Based on a one-foot image of a measurement subject read by the image reader 50, the toe estimation unit 58 estimates the toe position. For example, the toe estimation unit 58 may binarize an arbitrary region on the toe side of the one-foot image 70 and detect the contour between the region occupied by the one foot and the region occupied by the white paper 80. In the contour thus detected, a point located on the most toe side may be estimated to be the toe position. In this case, the one-foot image 70 is an image captured from the medial side or lateral side of one foot such that the toe is present on the contour of the captured one foot.

The foot length calculation unit 60 calculates the foot length of the measurement subject based on the toe position estimated by the toe estimation unit 58 and the heel point position estimated by the heel estimation unit 56. In specific, when the foot length directions in the one-foot image 70, i.e., the left and right directions in Fig. 4A, are defined as X directions, using $X_t$ as the X coordinate of the estimated toe position and $X_h$ as the X coordinate of the estimated heel point position, the foot length calculation unit 60 can calculate, as the foot length, the absolute value of the difference between $X_t$ and $X_h$. At the time, the foot length may also be calculated based on comparison with the reference object shown in the one-foot image 70.

The foot length correction unit 62 corrects the foot length calculated by the foot length calculation unit 60. For example, the foot length correction unit 62 corrects the foot length calculated by the foot length calculation unit, based on a multiple regression model using a size of and an angle at a predetermined position of the reference object in the one-foot image, a parameter based on information on the actual size of the reference object and information on the actual angle at the predetermined position, and a coefficient based on the type of the contour shape. The correction processing performed by the foot length correction unit 62 will be detailed later.

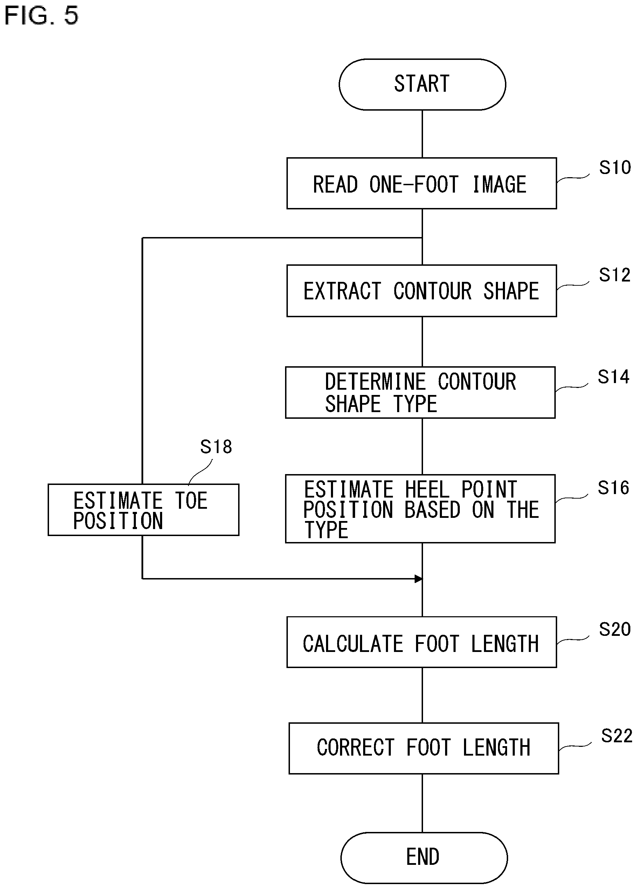

Fig. 5 is a flowchart that shows an example of processing performed by the heel position estimation server 20. The image reader 50 reads a one-foot image received by the communication unit 40 from the user terminal 10 (S10). From the one-foot image of a measurement subject read by the image reader 50, the contour extractor 52 extracts a contour shape of a foot part that includes at least part of a heel portion (S12). The judgment unit 54 determines the type of the contour shape extracted by the contour extractor 52 (S14). Based on the type of the contour shape determined by the judgment unit 54, the heel estimation unit 56 estimates the heel point position in the contour shape (S16). After the step S10, in parallel with the steps S12-S16, the toe estimation unit 58 estimates the toe position based on the one-foot image of the measurement subject read by the image reader 50 (S18). The foot length calculation unit 60 calculates the foot length of the measurement subject based on the toe position estimated by the toe estimation unit 58 at the step S18 and the heel point position estimated by the heel estimation unit 56 at the step S16 (S20). The foot length correction unit 62 corrects the foot length calculated by the foot length calculation unit 60 (S22), and the processing is terminated.

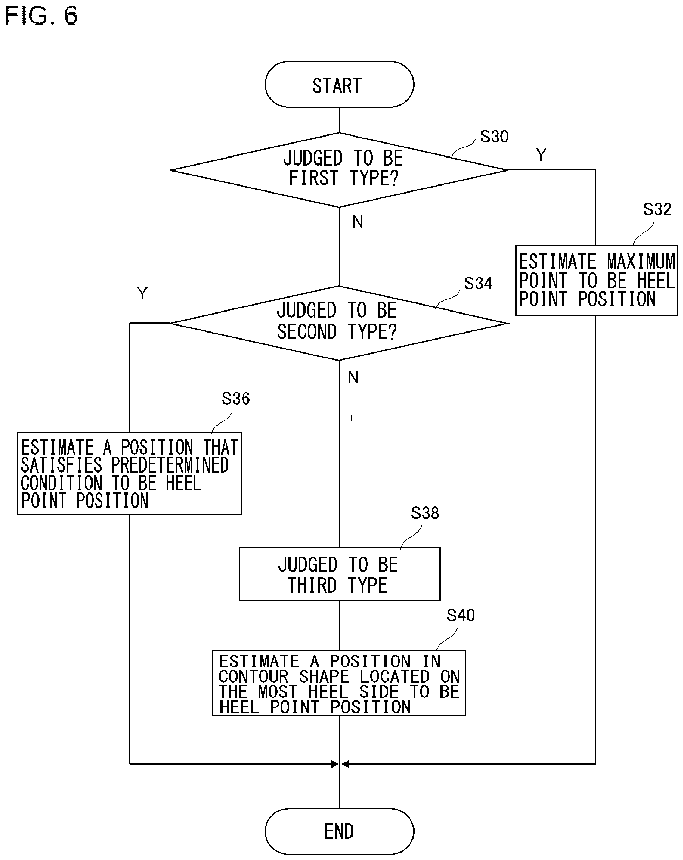

Fig. 6 is a flowchart that specifically shows the processing performed by the judgment unit 54 at the step S14 and the processing performed by the heel estimation unit 56 at the step S16 shown in Fig. 5, in the example of the operations performed by the heel position estimation server 20. The judgment unit 54 judges whether or not the contour shape is a first type having a maximum point when the heel side in a foot length direction of the one-foot image is defined as the positive direction (S30). When the judgment unit 54 judges the contour shape to be the first type (Y at S30), the heel estimation unit 56 estimates the maximum point in the contour shape to be the heel point position (S32).

When the judgment unit 54 judges the contour shape to be not the first type (N at S30), the judgment unit 54 judges whether or not the contour shape is a second type in which the magnitude of an inclination of the contour shape with respect to a foot length direction or the degree of a change in the inclination satisfies a predetermined condition (S34). When the judgment unit 54 judges the contour shape to be the second type (Y at S34), a position that satisfies the predetermined condition is estimated to be the heel point position (S36).

When the judgment unit 54 judges the contour shape to be not the second type (N at S34), the judgment unit 54 judges that the contour shape is a third type (S38). When the judgment unit 54 judges the contour shape to be the third type (S38), the heel estimation unit 56 estimates a position in the contour shape located on the most heel side in a foot length direction to be the heel point position (S40).

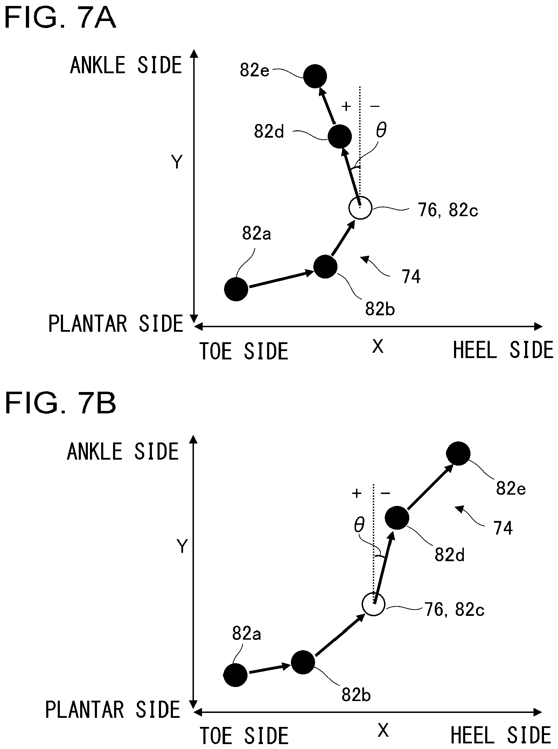

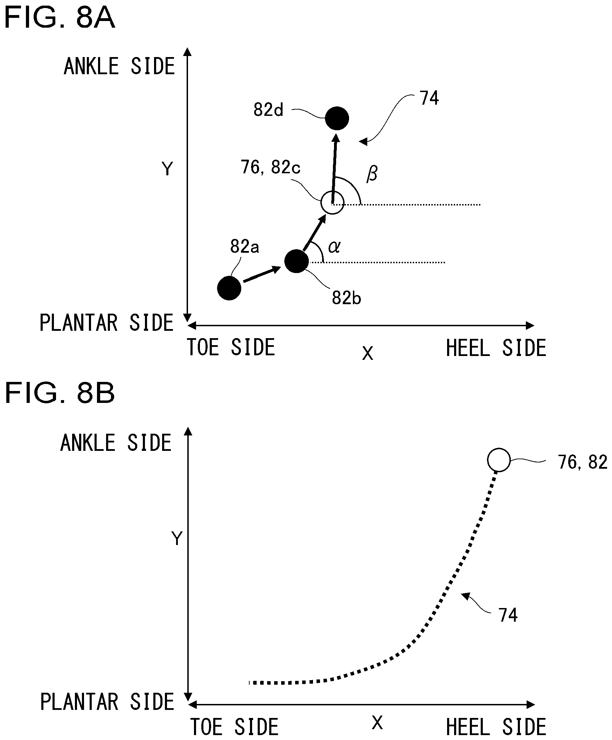

Figs. 7A, 7B, 8A and 8B show examples of each type of the contour shape 74 judged by the judgment unit 54. Although Figs. 7A, 7B, 8A and 8B show the contour shape 74 as a set of points, this does not mean that the data format of the contour shape 74 is limited to the raster format. Even when the data of the contour shape 74 is in the vector format, the contour shape 74 can be seen as a set of points calculated at predetermined intervals in a two-dimensional coordinate system based on the vector format data. In Figs. 7A, 7B, and 8A, the points constituting the contour shape 74 are indicated as contour points 82a-82e. When the contour points 82a-82e are not distinguished from each other, they may be collectively referred to as contour points 82. Also, the contour points 82a-82e in each drawing are provided to distinguish the contour points 82 in the drawing and do not indicate the same contour points 82 in different drawings even though they have the same reference characters.

Fig. 7A shows an example of the contour shape 74 judged to be the first type. Fig. 7A schematically shows a magnified view of part of the entire contour shape 74 located on the most heel side in a foot length direction of the one-foot image 70. The judgment unit 54 selects the contour points 82 constituting the contour shape 74 two by two from the plantar side toward the ankle side. At that time, the judgment unit 54 selects the contour points 82 such that the contour point 82 on the ankle side of the two contour points 82 selected earlier coincides with the contour point 82 on the plantar side of the two contour points 82 selected immediately thereafter. The judgment unit 54 sequentially calculates an angle $\theta$ between the vector from the contour point 82 on the plantar side to the contour point 82 on the ankle side thus selected and a straight line parallel to the direction from the plantar side to the ankle side (Y-axis direction). For example, the judgment unit 54 calculates the angle $\theta$ between the vector from the contour point 82a to the contour point 82b and a straight line parallel to the Y-axis and then calculates the angle $\theta$ between the vector from the contour point 82b to the contour point 82c and a straight line parallel to the Y-axis. The sign of the angle $\theta$ is positive when the vector is directed toward the toe side with respect to a straight line parallel to the Y-axis, and the sign is negative when the vector is directed toward the heel side with respect to a straight line parallel to the Y-axis.

When the sign of the angle $\theta$ becomes positive for the first time, i.e., when the vector from the contour point 82 on the plantar side to the contour point 82 on the ankle side selected is directed toward the toe side with respect to a straight line parallel to the Y-axis, the judgment unit 54 judges the contour shape 74 to be the first type. The heel estimation unit 56 then estimates the contour point 82 on the plantar side of the two contour points 82 selected at the time, to be the position of a heel point 76. In the example shown in Fig. 7A, the vector from the contour point 82c to the contour point 82d is directed toward the toe side with respect to a straight line parallel to the Y-axis, so that the contour point 82c is estimated to be the position of the heel point 76. In other words, the judgment unit 54 judges the contour shape 74 to be the first type when the contour shape 74 has a maximum point when the heel side in a foot length direction of the one-foot image 70 is defined as the positive direction. The heel estimation unit 56 then estimates the maximum point to be the heel point position. When there are multiple contour points 82 that satisfy the aforementioned condition, the heel estimation unit 56 may estimate the contour point 82 located on the most plantar side to be the position of the heel point 76.

When the sign of the angle $\theta$ becomes positive for the first time and when the angle $\theta$ exceeds a predetermined upper limit, the judgment unit 54 may not judge the contour shape to be the first type and may proceed to the selection of the next contour point 82. This can reduce the cases where the heel point position is estimated incorrectly because of noise or the like in the one-foot image 70, for example. The upper limit of the angle $\theta$ may be 45 degrees, for example.

Fig. 7B shows an example of the contour shape 74 judged to be the second type. Fig. 7B schematically shows a magnified view of part of the entire contour shape 74 located continuously from the position on the most plantar side toward the heel side of the one-foot image 70. After judging the contour shape 74 to be not the first type, the judgment unit 54 selects the contour points 82 constituting the contour shape 74 two by two from the plantar side toward the ankle side, as previously described. The judgment unit 54 sequentially calculates the angle $\theta$ between the vector from the contour point 82 on the plantar side to the contour point 82 on the ankle side thus selected and a straight line parallel to the direction from the plantar side to the ankle side (Y-axis direction).

When the sign of the angle $\theta$ remains negative and when the angle $\theta$ is a predetermined angle or larger, i.e., the absolute value of the angle $\theta$ is less than or equal to the absolute value of the predetermined angle, the judgment unit 54 may judge the contour shape 74 to be the second type. The heel estimation unit 56 then estimates the contour point 82 on the plantar side of the two contour points 82 selected at the time, to be the position of the heel point 76. In the example shown in Fig. 7B, the angle $\theta$ between the vector from the contour point 82c to the contour point 82d and a straight line parallel to the Y-axis is larger than or equal to the predetermined angle, so that the contour point 82c is estimated to be the position of the heel point 76. The predetermined angle in this case may be -5 degrees, for example. In other words, when the contour shape 74 includes a position at which the magnitude of the inclination with respect to a foot length direction (an X-axis direction) is a predetermined threshold or greater, the judgment unit 54 judges the contour shape 74 to be the second type. The predetermined threshold in this case is a value obtained by subtracting the abovementioned predetermined angle from 90 degrees and may be 85 degrees, for example. When there are multiple contour points 82 that satisfy the aforementioned condition, the heel estimation unit 56 may estimate the contour point 82 located on the most plantar side to be the position of the heel point 76.

Fig. 8A shows another example of the contour shape 74 judged to be the second type. Fig. 8A schematically shows a magnified view of part of the entire contour shape 74 located continuously from the position on the most plantar side toward the heel side of the one-foot image 70. After judging the contour shape 74 to be not the first type, the judgment unit 54 selects the contour points 82 constituting the contour shape 74 two by two from the plantar side toward the ankle side, as previously described. The judgment unit 54 sequentially calculates an angle $\Theta 2$, not illustrated, between the vector from the contour point 82 on the plantar side to the contour point 82 on the ankle side thus selected and a straight line parallel to a foot length direction (an X-axis direction).

When the angle $\Theta 2$ for the selected two contour points 82 falls within a first inclination range $\alpha$ and when the angle $\Theta 2$ for the two contour points 82 subsequently selected falls within a second inclination range $\beta$, the judgment unit 54 judges the contour shape to be the second type. The heel estimation unit 56 then estimates the contour point 82 on the plantar side of the two contour points 82 selected later, i.e., the contour point 82 on the ankle side of the two contour points 82 selected earlier, to be the position of the heel point 76. In the example shown in Fig. 8A, the angle $\Theta 2$ between the vector from the contour point 82b to the contour point 82c and a straight line parallel to the X-axis falls within the first inclination range $\alpha$, and the angle $\Theta 2$ between the vector from the contour point 82c to the contour point 82d and a straight line parallel to the X-axis falls within the second inclination range $\beta$, so that the contour point 82c is estimated to be the position of the heel point 76. The second inclination range $\beta$ is greater than the first inclination range $\alpha$ by a predetermined amount of inclination. For example, the first inclination range $\alpha$ may be greater than 45 degrees and less than or equal to 85 degrees, and the second inclination range $\beta$ may be greater than 85 degrees and less than or equal to 90 degrees. When there are multiple contour points 82 that satisfy the aforementioned condition, the heel estimation unit 56 may estimate the contour point 82 located on the most plantar side to be the position of the heel point 76.

The judgment unit 54 may perform the judgment for the second type described with reference to Fig. 7B and the judgment for the second type described with reference to Fig. 8A, selectively or in stages. That is, the judgment unit 54 may perform only one of the judgment for the second type described with reference to Fig. 7B and the judgment for the second type described with reference to Fig. 8A or may perform, when the contour shape is not judged to be the second type in one of the judgments, the other judgment.

Fig. 8B shows an example of the contour shape 74 judged to be the third type. When the judgment unit 54 judges the contour shape 74 to be not the first type or the second type, the judgment unit 54 judges the contour shape 74 to be the third type. In this case, as shown in Fig. 8B, the heel estimation unit 56 estimates the contour point 82 at a position in the contour shape 74 located on the most heel side in a foot length direction (an X direction) to be the position of the heel point 76.

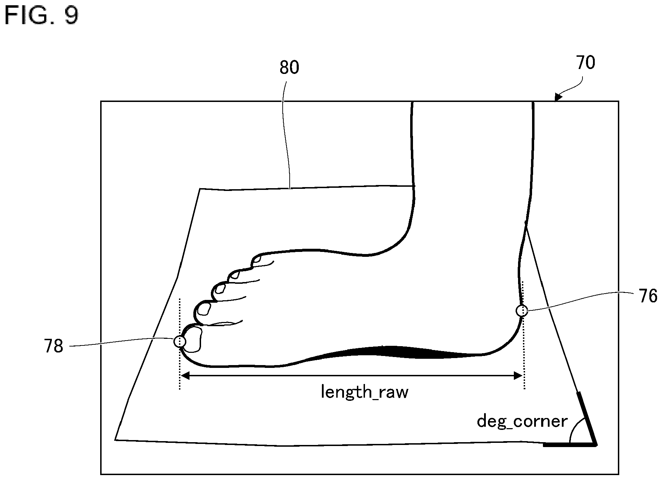

Fig. 9 is a schematic diagram that shows foot length correction performed by the foot length correction unit 62. The foot length calculated by the foot length calculation unit 60 is likely to include an error due to the capturing angle of the one-foot image 70 or the like. In particular, in the present embodiment, the heel point position estimated based on the type of the contour shape 74 that has been determined is different in a direction perpendicular to a foot length direction and is therefore easily affected by the capturing angle of the one-foot image 70. Therefore, the foot length correction unit 62 corrects the foot length calculated by the foot length calculation unit 60, based on a multiple regression model using a length along a foot length direction of the white paper 80, which is the reference object in the one-foot image 70, an angle of a corner of the white paper 80, a parameter based on information on the actual length along a foot length direction of the white paper 80 and information on the actual angle of the corner of the white paper 80, and a coefficient based on the type of the contour shape 74.

The foot length (length_raw) of the one foot in the one-foot image 70 is calculated based on the coordinates of the toe 78 and the coordinates of the heel point 76. The foot length (length_raw) is standardized based on the length along a foot length direction of the white paper 80 in the one-foot image 70 and information on the actual length along a foot length direction of the white paper 80, so that a standardized foot length (Xstd_length_raw) is calculated. Also, the angle (deg_corner) of a corner of the white paper 80 in the one-foot image 70 is calculated by contour extraction or the like. The corner of the white paper 80 may be an arbitrary corner of the four corners of the white paper 80; in the example shown in Fig. 9, the lower right corner is selected. The angle (deg_corner) of a corner of the white paper 80 is standardized using the actual angle of the corner of the white paper 80, such as the angle in plan view of the white paper 80, i.e., 90 degrees, so that a standardized angle (Xstd_deg_corner) is calculated.

A multiple regression equation in which the standardized foot length (Xstd_length_raw) and the standardized angle (Xstd_deg_corner) are set as explanatory variables and a foot length after correction (Ylength) is set as the objective variable is expressed by the following equation (1).

$$Ylength = a*Xstd\_length\_raw + b*Xstd\_deg\_corner + c \quad (1)$$

where each of a, b, and c is a coefficient and can be made different for each type of the contour shape 74

As described above, according to the present embodiment, the contour extractor 52 extracts the contour shape 74 of a foot part that includes at least part of a heel portion, from the one-foot image 70 of a measurement subject. The judgment unit 54 determines the type of the contour shape 74. Based on the type of the contour shape thus determined, the heel estimation unit 56 estimates the heel point position in the contour shape. Therefore, the heel position estimation server 20 can appropriately estimate the heel point position, with reduced influence of the capturing conditions of the one-foot image 70 or the individual differences in the three-dimensional shape of one foot.

Also, according to the present embodiment, the judgment unit 54 judges whether or not the contour shape 74 falls into the first type having a maximum point when the heel side in a foot length direction of the one-foot image 70 is defined as the positive direction. When the contour shape 74 is judged to be the first type, the heel estimation unit 56 estimates the maximum point to be the position of the heel point 76. Accordingly, in the case of a standard heel shape such that the position of the heel point 76 corresponds to a maximum point on the heel side, the heel position estimation server 20 can appropriately estimate the position of the heel point 76.

Also, according to the present embodiment, when the judgment unit 54 has judged the contour shape to be not the first type, the judgment unit 54 judges whether or not the contour shape 74 falls into the second type in which the magnitude of an inclination of the contour shape 74 with respect to a foot length direction or the degree of a change in the inclination satisfies a predetermined condition. When the contour shape 74 is judged to be the second type, the heel estimation unit 56 estimates a position in the contour shape 74 that satisfies the predetermined condition to be the position of the heel point 76. Accordingly, even when the contour shape 74 has no maximum point on the heel side, the heel position estimation server 20 can appropriately estimate the position of the heel point 76.

Also, according to the present embodiment, when the inclination of the contour shape 74 with respect to a foot length direction changes, along the contour shape 74 from the plantar side toward the heel side in a foot length direction, from the first inclination range $\alpha$ to the second inclination range $\beta$, which is greater than the first inclination range $\alpha$ by a predetermined amount of inclination, the judgment unit 54 judges the contour shape 74 to be the second type. When the contour shape 74 is judged to be the second type, the heel estimation unit 56 estimates a position in the contour shape 74 at which the inclination changes from the first inclination range $\alpha$ to the second inclination range $\beta$, to be the position of the heel point 76. Accordingly, based on the change in inclination of the contour shape 74, the heel position estimation server 20 can appropriately estimate the position of the heel point 76.

Also, according to the present embodiment, when the contour shape 74 includes a position at which the magnitude of the inclination with respect to a foot length direction is a predetermined threshold or greater, the judgment unit 54 judges the contour shape 74 to be the second type. When the contour shape 74 is judged to be the second type, the heel estimation unit 56 estimates a position in the contour shape 74 at which the magnitude of the inclination is greater than or equal to the threshold and that is located on the most plantar side, to be the position of the heel point 76. Accordingly, based on the magnitude of the inclination of the contour shape 74, the heel position estimation server 20 can appropriately estimate the position of the heel point 76.

Also, according to the present embodiment, when the judgment unit 54 has judged the contour shape 74 to be not the first type or the second type, the judgment unit 54 judges the contour shape 74 to be the third type. When the contour shape 74 is judged to be the third type, the heel estimation unit 56 estimates a position in the contour shape 74 located on the most heel side in a foot length direction to be the position of the heel point 76. Accordingly, even when the contour shape 74 cannot be classified in the first type or the second type, the heel position estimation server 20 can appropriately estimate the position of the heel point 76 within the range where the contour shape 74 has been extracted.

The embodiment described above may be a program for causing a computer to perform functions to realize the method described above or may be a storage medium storing the program. The storage medium storing such a program may be a non-transitory and tangible computer readable storage medium and may be a non-volatile memory, a magnetic storage medium such as a magnetic tape or a magnetic disk, or an optical storage medium such as an optical disk.

The present disclosure has been described based on an embodiment. The embodiment is intended to be illustrative only, and it will be obvious to those skilled in the art that various modifications to a combination of constituting elements or processes could be developed and that such modifications also fall within the scope of the present disclosure.

Also, when the embodiment set forth above is generalized, the following aspects are obtained.

(1) A heel position estimation device according to one aspect of the present disclosure includes:

a contour extractor that extracts, from a one-foot image of a measurement subject, a contour shape of a foot part that includes at least part of a heel portion;
a judgment unit that determines the type of the contour shape; and
a heel estimation unit that estimates a heel point position in the contour shape, based on the type of the contour shape determined.

(2) In the heel position estimation device described in (1),

the judgment unit may judge whether or not the contour shape is a first type having a maximum point when a heel side in a foot length direction of the one-foot image is defined as the positive direction, and
when the contour shape is judged to be the first type, the heel estimation unit may estimate the maximum point to be the heel point position.

(3) In the heel position estimation device described in (2),

when the judgment unit has judged the contour shape to be not the first type, the judgment unit may judge whether or not the contour shape is a second type in which the magnitude of an inclination of the contour shape with respect to the foot length direction or the degree of a change in the inclination satisfies a predetermined condition, and
when the contour shape is judged to be the second type, the heel estimation unit may estimate a position in the contour shape that satisfies the predetermined condition to be the heel point position.

(4) In the heel position estimation device described in (3),

when the inclination of the contour shape with respect to the foot length direction changes, along the contour shape from a plantar side toward the heel side in the foot length direction, from a first inclination range to a second inclination range, which is greater than the first inclination range by a predetermined amount of inclination, the judgment unit may judge the contour shape to be the second type, and
when the contour shape is judged to be the second type, the heel estimation unit may estimate a position in the contour shape at which the inclination changes from the first inclination range to the second inclination range, to be the heel point position.

(5) In the heel position estimation device described in (3),

when the contour shape includes a position at which the magnitude of the inclination with respect to the foot length direction is a predetermined threshold or greater, the judgment unit may judge the contour shape to be the second type, and
when the contour shape is judged to be the second type, the heel estimation unit may estimate a position in the contour shape at which the magnitude of the inclination is greater than or equal to the threshold and that is located on the most plantar side, to be the heel point position.

(6) In the heel position estimation device described in any one of (3) through (5),

when the judgment unit has judged the contour shape to be not the first type or the second type, the judgment unit may judge the contour shape to be a third type, and
when the contour shape is judged to be the third type, the heel estimation unit may estimate a position in the contour shape located on the most heel side in the foot length direction to be the heel point position.

(7) The heel position estimation device described in any one of (1) through (6) may further include:

a toe estimation unit that estimates a toe position from the one-foot image; and
a foot length calculation unit that calculates a foot length based on the toe position estimated and the heel point position estimated.

(8) The heel position estimation device described in (7) may further include a foot length correction unit that corrects a foot length calculated by the foot length calculation unit, based on a multiple regression model using a size of a reference object in the one-foot image and an angle of a predetermined portion of the reference object, a parameter based on information on the actual size of the reference object and information on the actual angle of the predetermined portion of the reference object, and a coefficient based on the type of the contour shape.

(9) A heel position estimation method according to one aspect of the present disclosure includes:

extracting, from a one-foot image of a measurement subject, a contour shape of a foot part that includes at least part of a heel portion;
determining the type of the contour shape; and
estimating a heel point position in the contour shape, based on the type of the contour shape determined.

(10) A program according to one aspect of the present disclosure causes a computer to implement:

extracting, from a one-foot image of a measurement subject, a contour shape of a foot part that includes at least part of a heel portion;
determining the type of the contour shape; and
estimating a heel point position in the contour shape, based on the type of the contour shape determined.

**Claims**

1. A heel position estimation device, comprising:

   a contour extractor (52) that is configured to extract, from a one-foot image of a measurement subject, a contour shape of a foot part that includes at least part of a heel portion;
   a judgment unit (54) that is configured to determine the type of the contour shape; and
   a heel estimation unit (56) that is configured to estimate a heel point position in the contour shape, based on the type of the contour shape determined.

2. The heel position estimation device according to claim 1,

   wherein the judgment unit (54) is configured to judge whether the contour shape is a first type having a maximum point when a heel side in a foot length direction of the one-foot image is defined as the positive direction, and
   wherein, when the contour shape is judged to be the first type, the heel estimation unit (56) is configured to estimate the maximum point to be the heel point position.

3. The heel position estimation device according to claim 2,

   wherein, when the judgment unit (54) has judged the contour shape to be not the first type, the judgment unit (54) is configured to judge whether the contour shape is a second type in which the magnitude of an inclination of the contour shape with respect to the foot length direction or the degree of a change in the inclination satisfies a predetermined condition, and
   wherein, when the contour shape is judged to be the second type, the heel estimation unit (56) is configured to estimate a position in the contour shape that satisfies the predetermined condition to be the heel point position.

4. The heel position estimation device according to claim 3,

   wherein, when the inclination of the contour shape with respect to the foot length direction changes, along the contour shape from a plantar side toward the heel side in the foot length direction, from a first inclination range to a second inclination range, which is greater than the first inclination range by a predetermined amount of inclination, the judgment unit (54) is configured to judge the contour shape to be the second type, and
   wherein, when the contour shape is judged to be the second type, the heel estimation unit (56) is configured to estimate a position in the contour shape at which the inclination changes from the first inclination range to the second inclination range, to be the heel point position.

5. The heel position estimation device according to claim 3,

wherein, when the contour shape includes a position at which the magnitude of the inclination with respect to the foot length direction is a predetermined threshold or greater, the judgment unit (54) is configured to judge the contour shape to be the second type, and

wherein, when the contour shape is judged to be the second type, the heel estimation unit (56) is configured to estimate a position in the contour shape at which the magnitude of the inclination is greater than or equal to the threshold and that is located on the most plantar side, to be the heel point position.

6. The heel position estimation device according to any one of claims 3 through 5,

wherein, when the judgment unit (54) has judged the contour shape to be not the first type or the second type, the judgment unit (54) is configured to judge the contour shape to be a third type, and

wherein, when the contour shape is judged to be the third type, the heel estimation unit (56) is configured to estimate a position in the contour shape located on the most heel side in the foot length direction to be the heel point position.

7. The heel position estimation device according any one of claims 1 through 6, further comprising:

a toe estimation unit (58) that is configured to estimate a toe position from the one-foot image; and

a foot length calculation unit (60) that is configured to calculate a foot length based on the toe position estimated and the heel point position estimated.

8. The heel position estimation device according to claim 7, further comprising a foot length correction unit (62) that is configured to correct a foot length calculated by the foot length calculation unit, based on a multiple regression model using a size of a reference object in the one-foot image and an angle of a predetermined portion of the reference object, a parameter based on information on the actual size of the reference object and information on the actual angle of the predetermined portion of the reference object, and a coefficient based on the type of the contour shape.

9. A heel position estimation method, comprising:

extracting, from a one-foot image of a measurement subject, a contour shape of a foot part that includes at least part of a heel portion;

determining the type of the contour shape; and

estimating a heel point position in the contour shape, based on the type of the contour shape determined.

10. A program causing a computer to implement:

extracting, from a one-foot image of a measurement subject, a contour shape of a foot part that includes at least part of a heel portion;

determining the type of the contour shape; and

estimating a heel point position in the contour shape, based on the type of the contour shape determined.

FIG. 1

FIG. 2

USER TERMINAL 10

- IMAGE ACQUIRER 30
- DISPLAY UNIT 32
- COMMUNICATION UNIT 34
- OPERATION PROCESSING UNIT 36

100

18

HEEL POSITION ESTIMATION SERVER 20

- COMMUNICATION UNIT 40
- COMPUTING UNIT 42
- OUTPUT UNIT 46
- STORAGE UNIT 44

## FIG. 3

42

COMPUTING UNIT

50

IMAGE READER

52

CONTOUR EXTRACTOR

54

JUDGMENT UNIT

56

HEEL ESTIMATION UNIT

58

TOE ESTIMATION UNIT

60

FOOT LENGTH
CALCULATION UNIT

62

FOOT LENGTH
CORRECTION UNIT

FIG. 4A

80

70

LATERAL SIDE

MEDIAL SIDE

FOOT LENGTH DIRECTION

72

FIG. 4B

72

74

FIG. 5

```
                          ┌──────────┐
                          │  START   │
                          └────┬─────┘
                               │
                  ┌────────────────────────┐   S10
                  │   READ ONE-FOOT IMAGE  │
                  └────────────┬───────────┘
                               │
                  ┌────────────────────────┐   S12
                  │  EXTRACT CONTOUR SHAPE │
                  └────────────┬───────────┘
                               │
                  ┌────────────────────────┐   S14
                  │   DETERMINE CONTOUR    │
                  │   SHAPE TYPE           │
                  └────────────┬───────────┘
                               │
   S18                         │
 ┌────────────────┐  ┌────────────────────────┐   S16
 │ ESTIMATE TOE   │  │  ESTIMATE HEEL POINT   │
 │ POSITION       │  │  POSITION BASED ON THE │
 └───────┬────────┘  │  TYPE                  │
         │           └────────────┬───────────┘
         └────────────────────────┤
                               │
                  ┌────────────────────────┐   S20
                  │  CALCULATE FOOT LENGTH │
                  └────────────┬───────────┘
                               │
                  ┌────────────────────────┐   S22
                  │  CORRECT FOOT LENGTH   │
                  └────────────┬───────────┘
                               │
                          ┌──────────┐
                          │   END    │
                          └──────────┘
```

FIG. 6

```
                    ┌──────────────┐
                    │    START     │
                    └──────┬───────┘
                           │
                    ╱──────┴──────╲        S30
                   ╱   JUDGED TO BE  ╲──────────── Y ──────┐
                   ╲   FIRST TYPE?   ╱                     │
                    ╲──────┬──────╱                        │
                           │ N                             │  S32
                           │                    ┌──────────┴──────────┐
                           │                    │ ESTIMATE MAXIMUM    │
                           │                    │ POINT TO BE HEEL    │
                           │                    │ POINT POSITION      │
                           │                    └──────────┬──────────┘
                    ╱──────┴──────╲        S34              │
         ┌── Y ────╱   JUDGED TO BE  ╲─────                 │
         │         ╲   SECOND TYPE?  ╱                      │
         │          ╲──────┬──────╱                         │
         │                 │ N                              │
  S36    │                 │                                │
┌────────┴────────┐        │                                │
│ ESTIMATE A      │        │                                │
│ POSITION THAT   │        │                                │
│ SATISFIES       │        │                                │
│ PREDETERMINED   │        │                                │
│ CONDITION TO BE │        │                                │
│ HEEL POINT      │        │           S38                  │
│ POSITION        │  ┌─────┴────────┐                       │
└────────┬────────┘  │ JUDGED TO BE │                       │
         │           │ THIRD TYPE   │                       │
         │           └─────┬────────┘                       │
         │                 │           S40                  │
         │        ┌────────┴──────────┐                     │
         │        │ ESTIMATE A        │                     │
         │        │ POSITION IN       │                     │
         │        │ CONTOUR SHAPE     │                     │
         │        │ LOCATED ON THE    │                     │
         │        │ MOST HEEL SIDE TO │                     │
         │        │ BE HEEL POINT     │                     │
         │        │ POSITION          │                     │
         │        └────────┬──────────┘                     │
         │                 │                                │
         └─────────────────┼────────────────────────────────┘
                           │
                    ┌──────┴───────┐
                    │     END      │
                    └──────────────┘
```

FIG. 7A

FIG. 7B

FIG. 8A

ANKLE SIDE

82d

74

76, 82c

β

Y

α

82a

82b

PLANTAR SIDE

TOE SIDE

X

HEEL SIDE

FIG. 8B

ANKLE SIDE

76, 82

Y

74

PLANTAR SIDE

TOE SIDE

X

HEEL SIDE

FIG. 9

Europäisches
Patentamt

European
Patent Office

Office européen
des brevets

# EUROPEAN SEARCH REPORT

**Application Number**

EP 24 18 1811

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 106 127 773 A (BEIJING 3D WORLD TECH CO LTD) 16 November 2016 (2016-11-16) * sections "Summary of the invention", "Detailed description of the invention" * | 1-10 | INV. G06T7/62 G06T7/73 |
| A | CHUN SUNGKUK ET AL: "A Foot-Arch Parameter Measurement System Using a RGB-D Camera", SENSORS, vol. 17, no. 8, 4 August 2017 (2017-08-04) , page 1796, XP093028245, DOI: 10.3390/s17081796 * abstract * * section 4.4.1 * * Algorithm 2 * * figures 1a, 7b, 7c * | 1-10 | |
| A | US 5 025 476 A (GOULD NATHANIEL [US] ET AL) 18 June 1991 (1991-06-18) * abstract * * column 7, line 50 - line 65 * * column 8, line 28 - line 65 * * figure 5 * | 1-10 | |
| A | CN 108 805 138 B (BEIJING METRICA TECH CO LTD) 22 April 2022 (2022-04-22) * section "Summary of the invention" * * figures 9, 12-16 * | 1-10 | TECHNICAL FIELDS SEARCHED (IPC) G06T |

The present search report has been drawn up for all claims

| Place of search | Date of completion of the search | Examiner |
|---|---|---|
| Munich | 30 October 2024 | Engels, Angela |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 1811

This annex lists the patent family members relating to the patent documents cited in the above-mentioned European search report.
The members are as contained in the European Patent Office EDP file on
The European Patent Office is in no way liable for these particulars which are merely given for the purpose of information.

30-10-2024

| Patent document cited in search report | | Publication date | Patent family member(s) | | Publication date |
|---|---|---|---|---|---|
| CN 106127773 | A | 16-11-2016 | NONE | | |
| US 5025476 | A | 18-06-1991 | AU | 4428989 A | 28-05-1990 |
| | | | CA | 2001429 A1 | 30-04-1990 |
| | | | CN | 1042296 A | 23-05-1990 |
| | | | KR | 900702476 A | 07-12-1990 |
| | | | US | 5025476 A | 18-06-1991 |
| | | | WO | 9005345 A1 | 17-05-1990 |
| CN 108805138 | B | 22-04-2022 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019045463 A